# EUROPÄISCHE PATENTSCHRIFT

(11) **EP 1 048 646 B1**
(45) Veröffentlichungstag und Bekanntmachung des Hinweises auf die Patenterteilung: **04.07.2001**
(21) Anmeldenummer: 99107704.1
(22) Anmeldetag: 17.04.1999
(51) Int. Cl.: C07C 231/24, C07D 207/267, C07C 233/03, C07D 207/26

(54) **Verfahren zur kontinuierlichen Destillation von thermolabilen Monomeren**
Process for the continuous distillation of thermolabile monomers
Procédé pour la distillation en continu de monomères thermolabiles

(43) Veröffentlichungstag der Anmeldung: 02.11.2000
(73) Patentinhaber: BASF AKTIENGESELLSCHAFT, 67056 Ludwigshafen (DE)
(72) Erfinder: Dupuis, Jacques Dr., 67069 Ludwigshafen (DE); Winter, Manfred, 67596 Dittelsheim-Hessloch (DE); Kröner, Michael Dr., 14558 Bergholz-Rehbrücke (DE)

(56) Entgegenhaltungen:
- EP-A- 0 184 074
- EP-A- 0 231 901
- EP-A- 0 703 219
- DE-A- 19 711 925

## Beschreibung

Die Erfindung betrifft ein Verfahren zur kontinuierlichen Destillation von N-Vinylcarbonsäureamiden unter vermindertem Druck in Gegenwart von Formamid in einer Kolonne.

Aus der EP-A-0 231 901 ist ein Verfahren zur Reinigung von N-Vinylformamid durch fraktionierte Destillation des N-Vinylformamids bekannt. N-Vinylformamid ist ein thermolabiles Monomer. Es wird nach dem bekannten Verfahren in Gegenwart von Formamid unter einem Druck von 0,5 bis 30 mbar in einer Kolonne fraktioniert destilliert, wobei man die Destillation so steuert, daß das Destillat N-Vinylformamid mit einem Gehalt von 0,1 bis 15 Gew.-% Formamid enthält. Nach diesem Destillationsverfahren sind Monomerqualitäten erhältlich, aus denen Homopolymerisate von N- Vinylformamid mit sehr hohen Molmassen hergestellt werden können. Obwohl dieses Verfahren gegenüber der fraktionierten Destillation von N-Vinylformamid in Abwesenheit von Formamid entscheidende Vorteile in der Monomerqualität bietet, enthält das so hergestellte N-Vinylformamid noch immer Verunreinigungen im ppm-Bereich. Diese Verunreinigungen wurden bisher noch nicht identifiziert, sie machen sich jedoch störend bei der Polymerisation bemerkbar, weil sie zu einer Begrenzung der Molmasse der Polymerisate führen. die bei der Polymerisation störend wirken, im ppm-Bereich.

Aus der DE-A-197 11 925 ist ein Verfahren zur Reinigung von N-Vinylformamid bekannt, wobei man rohes N-Vinylformamid kontinuierlich in eine Fraktionierkolonne einspeist, um N-Vinylformamid mit hoher Reinheit zu erhalten. Die Destillation wird unter einem Druck am Kolonnenkopf von 0,1 bis 3 kPa durchgeführt.

Aus der EP-A-0 184 074 ist ein Verfahren zur Herstellung von NVinylformamid durch Pyrolyse von Formylalaninnitril in Gegenwart von Feststoffen als Katalysator unter vermindertem Druck bei Temperaturen von 250 bis 650 °C bekannt, wobei man die Pyrolyseprodukte bei einem Druck von 10 bis 50 mbar auf eine Temperatur von 200 bis -10°C abkühlt, so daß eine weitgehende fraktionierte Trennung in kondensiertes N-Vinylformamid und gasförmigen Cyanwasserstoff erfolgt. Der Cyanwasserstoff wird anschließend unter einem Druck von 5 bis 140 mbar einer Chemiesorption mit Acetaldehyd unter Bildung von Milchsäurenitril unterworfen.

Aus der EP-A-0 703 219 ist ein Verfahren zur Herstellung von N-Vinylverbindungen wie N-Vinylpyrrolidon und N-Vinyl-N-ethylacetamid durch thermische Zersetzung von N-(α-Acetoxyethyl)-Verbindungen bekannt. Die N-Vinylverbindungen werden durch Destillation unter vermindertem Druck aus dem Reaktionsgemisch isoliert.

Bei der Destillation von thermolabilen Monomeren entstehen häufig Spaltprodukte, die sich bei der Destillation anreichern und den Verlauf der Destillation beeinträchtigen. In einigen Fällen kann es dabei sogar zu einer Verstopfung der Destillationskolonne kommen. Andererseits können leichtsiedende Spaltprodukte von thermolabilen Monomeren als Verunreinigung im Destillat sein.

Der vorliegenden Erfindung liegt die Aufgabe zugrunde, ein verbessertes Verfahren zur Destillation von N-Vinylcarbonsäureamiden zur Verfügung zu stellen.

Die Aufgabe wird erfindungsgemäß gelöst mit einem Verfahren zurkontinuierlichen Destillation von N-Vinylcarbonsäureamiden in Gegenwart von Formamid in einer Kolonne, unter einem Druck, der am Kopt der Kolonne 0,1 bis 30 mbar beträgt, wenn man
- die N-Vinylcarbonsäureamiden getrennt von Formamid kontinuier lich in flüssiger Form in den unteren Teil der Kolonne bis zur Mitte der Kolonne zuführt,
- Formamid mit Hilfe eines Verdampfers am Kolonnensumpf verdampft und zusammen mit Bestandteilen, die höher als die N-Vinylcarbonsäureamiden sieden, aus dem Kolonnensumpf austrägt und durch Zugabe von frischem Formamid ersetzt,
- die N-Vinylcarbonsäureamiden in einem Seitenabzug im oberen Drittel der Kolonne abnimmt, wobei man die Destillation so steuert, daß die Monomeren weniger als 5 Gew.-% Formamid enthalten, und
- am Kopf der Kolonne einen Produktstrom abnimmt, der Bestandteile enthält, die einen niedrigeren Siedepunkt als die N-Vinylcarbonsäureamiden haben.

Unter thermolabilen Monomeren sollen monoethylenisch ungesättigte Monomere verstanden werden, die sich beim Destillieren zersetzen. Bei der Destillation von thermolabilen Monomeren entstehen häufig Verunreinigungen, die bereits im ppm-Bereich die Polymerisation der destillierten Monomeren stören. Zu solchen Monomeren gehören beispielsweise N-Vinylcarbonsäureamide. Sie können beispielsweise eine cyclische Struktur aufweisen wie N-Vinylpyrrolidon oder substituierte N-Vinylpyrrolidone oder sie haben eine offenkettige Struktur, die beispielsweise mit Hilfe der folgenden Formel beschrieben werden kann: 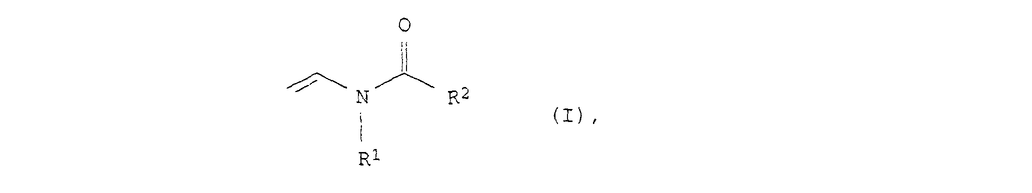 in der R¹ und R² gleich oder verschieden sein können und für Wasserstoff und C₁- bis C₆-Alkyl stehen. Monomere dieser Art sind z.B. N-Vinylformamid (R =R =H in Formel I), N-Vinyl-N-methylformamid, M-Vinylacetamid, N-Vinyl-N-methylacetamid, N-Vinyl-N-ethylacetamid, N-Vinyl-N-methylpropionamid und N-Vinylpropionamid. Das erfindungsgemäße Verfahren eignet sich insbesondere zur Herstellung von hochreinem N-Vinylformamid.

Bei dem erfindungsgemäßen Verfahren werden die thermolabilen Monomeren getrennt von Formamid kontinuierlich in flüssiger Form in den unteren Teil der Kolonne bis zur Mitte der Kolonne zugeführt.

Die zu reinigenden Monomeren können sowohl höher siedende als auch leichter siedende Bestandteile enthalten. So kann beispielsweise rohes N-Vinylformamid, das durch Pyrolyse von Formylalaninnitril hergestellt werden kann und bis zu 40 Gew.-% Formylalaninnitril enthält, nach dem erfindungsgemäßen Verfahren gereinigt werden. Die für das thermische Trennverfahren innerhalb der Kolonne notwendige Energie wird mit Hilfe eines Verdampfers am Kolonnensumpf durch Verdampfen von Formamid in das System eingebracht. Die Temperatur im Kolonnensumpf beträgt beispielsweise 70 bis 140, vorzugsweise 80 bis 110°C. Im Kolonnensumpf sammeln sich Bestandteile, die höher als die thermolabilen Monomeren sieden. Sie werden zusammen mit Formamid aus dem Kolonnensumpf kontinuierlich ausgetragen und kontinuierlich durch Zugabe von frischem Formamid ersetzt. Das Formamid kann gasförmig oder in flüssiger Form in den Verdampfer oder gegebenenfalls auch flüssig an einer Stelle bis zur Mitte der Kolonne dosiert werden. Vorzugsweise wird flüssiges Formamid in den Verdampfer gepumpt. Bezogen auf 1 Gew.-Teil der thermolabilen Monomeren setzt man beispielsweise 0,5 bis 5, vorzugsweise 1 bis 3 Gew.-Teile Formamid ein.

Das thermische Trennverfahren innerhalb der Kolonne erfolgt wie bei einer herkömmlichen Destillation. Die Zusammensetzung des Destillats hängt dabei im wesentlichen vom Rücklaufverhältnis und von der Anzahl der theoretische Böden in der Kolonne ab. Die Anzahl der theoretischen Böden der Kolonne beträgt beispielsweise 10 bis 60, vorzugsweise 25 bis 40. Das Rücklaufverhältnis wird beispielsweise in dem Bereich von 0,5 : 1 bis 4 : 1 eingestellt. Mit Hilfe der Einstellung des Rücklaufverhältnisses kann man beispielsweise den Gehalt an Formamid im Destillat leicht steuern.

Die thermolabilen Monomeren werden in einem Seitenabzug im oberen Drittel der Kolonne abgenommen, wobei die Destillation so gesteuert wird, daß die Monomeren weniger als 5 Gew.-% Formamid enthalten. Vorzugsweise fallen im Seitenabzug der Kolonne thermolabile Monomere mit einem Gehalt von weniger als 0,1 Gew.-% Formamid an. Besonders bevorzugt ist eine Ausführungsform des erfindungsgemäßen Verfahrens, bei der im Seitenabzug thermolabile Monomere abgenommen werden, die frei von Formamid sind. Die Temperatur des Kühlwassers für den Hauptkondensator wird so eingestellt, daß sie gerade ausreicht, um die thermolabilen Monomeren zu kondensieren, während die niedriger siedenden Verunreinigungen den Hauptkondensator gasförmig passieren und erst im Nachkondensator niedergeschlagen werden. Die Differenz zwischen der Temperatur des Kühlwassers für den Hauptkondensator und dem Siedepunkt des thermolabilen Monomeren unter den bei der Destillation herrschenden Druckbedingungen beträgt beispielsweise 1 bis 15, vorzugsweise 2 bis 12°C. Diejenigen Bestandteile, die am Hauptkondensator nicht kondensiert werden, werden am Kopf der Kolonne als Produktstrom abgenommen, der Verbindungen enthält, die einen niedrigeren Siedepunkt als die thermolabilen Monomeren haben. Die Kondensation des am Kopf der Kolonne abgezogenen Produktstroms erfolgt in einem Nachkondensator, der beispielsweise mit Sole gekühlt wird, die eine Temperatur von 0 bis -20°C hat. Der am Kopf der Kolonne abgetrennte Produktstrom enthält beispielsweise 10 bis 99,9, vorzugsweise 85 bis 98 Gew.-% an thermolabilen Monomeren. Die mit diesem Produktstrom abgetrennten Monomeren sind für eine Polymerisation ungeeignet und müssen entsorgt werden. Die Menge des am Kopf der Kolonne abgenommenen Produktstroms beträgt beispielsweise 0,1 bis 5, vorzugsweise 0,2 bis 2 Gew.-%, bezogen auf die bei der Destillation eingesetzten thermolabilen Monomeren.

Formamid wird vorzugsweise im Sumpf der Kolonne verdampft. Es kann jedoch auch außerhalb der Kolonne in einem Verdampfer verdampft und an einer Stelle zwischen dem Kolonnenboden und der Mitte der Kolonne in die Kolonne eingeleitet werden. Die thermolabilen Monomeren werden jedoch immer getrennt von Formamid in flüssiger Form oberhalb der Dosierstelle des gasförmigen Formamids in die Kolonne geleitet. Sie können gegebenenfalls außerhalb der Kolonne auf eine Temperatur bis zu ca. 5°C unterhalb ihres Siedepunkts unter den Destillationsbedingungen erhitzt werden.

Besonders bevorzugt wird nach dem erfindungsgemäßen Verfahren N-Vinylformamid gereinigt. Der Vorteil des Verfahrens gegenüber bekannten Verfahren zur Fraktionierung von N-Vinylformamid liegt vor allem darin, daß man Monomerqualitäten erhält, die zu besonders hochmolekularen Polymerisaten verarbeitet werden können. So erhält man beispielsweise aus erfindungsgemäß destilliertem N-Vinylformamid nach dem Verfahren der Wasser-in-Öl-Emulsionspolymerisation Poly-N-Vinylformamid mit K-Werten nach Fikentscher oberhalb von 240 (gemessen in 5 gew.-%iger wäßriger Kochsalzlösung bei 25°C, pH 7 und einer Polymerkonzentration von 0,1 Gew.-%). Die Herstellung von so hochmolekularen Polymerisaten von N-Vinylformamid ist schwierig, weil bereits Verunreinigungen, die im Bereich von einigen ppm liegen, die Polymerisation von N-Vinylformamid erheblich beeinflussen.

Schwersiedende Nebenprodukte werden bei dem erfindungsgemäßen Verfahren aus dem Kolonnensumpf zusammen mit dem kondensierten Formamid und gegebenenfalls geringen Mengen an thermolabilen Monomeren ausgetragen. Die Mengen an thermolabilen Monomeren im Kolonnensumpf betragen beispielsweise bis zu 2 Gew.-% und liegen vorzugsweise in dem Bereich von 0 bis 0,2 Gew.-%. Das Formamid kann aus dem Kolonnensumpf mit Hilfe einer fraktionierten Destillation zurückgewonnen und bei dem erfindungsgemäßen Verfahren erneut eingesetzt werden.

### Beispiel 1

Die Kolonne hatte einen Durchmesser von 200 mm und eine Länge von 5000 mm. Sie enthielt Gewebepackungen aus Edelstahl mit einer Oberfläche von 500 m²/m³. Die Füllung der Kolonne entsprach etwa 25 theoretischen Böden. Am Kolonnenkopf befand sich ein Hauptkondensator, der mit Wasser bei einer Temperatur von 50°C betrieben wurde. Der Nachkondensator am Kolonnenkopf wurde mit Wasser gekühlt, das eine Temperatur von 5°C hatte.

Im unteren Drittel der Kolonne wurden 14 kg/h rohes N-Vinylformamid mit einem Gehalt von ca. 31 % Formylalaninnitril, das auf eine Temperatur von 70°C vorgewärmt worden war, in die Kolonne eingebracht. Zum Wärmeeintrag dosierte man 19 kg/h dampfförmiges Formamid, das auf eine Temperatur von 100°C erhitzt worden war, in den Kolonnensumpf. Die Dosierung des gasförmigen Formamids erfolgte derart, daß die Regeltemperatur im unteren Drittel der Kolonne möglichst konstant blieb. Der Druck betrug am Kopf der Kolonne 3 mbar. Bei einem Rücklaufverhältnis von 2 : 1 erhielt man als Hauptkondensat, das in einem Seitenabzug im oberen Drittel der Kolonne ausgetragen wurde, 9,5 kg/h N-Vinylformamid mit einer Reinheit von >99 %. Es enthielt 0,08 Gew.-% Formamid.

Diejenigen Stoffe, die im Hauptkondensator nicht kondensiert werden konnten werden im Nachkondensator niedergeschlagen und separat ausgetragen. Im stationären Betrieb fielen im Sumpf bei einem Druck von 12 mbar und einer Temperatur von 97°C ca. 20 kg/h Produkt an. Das Sumpfprodukt enthielt <0,1 Gew.-% N-Vinylformamid.

### Beispiel 2

Für die kontinuierliche Destillation wurde eine Kolonne verwendet, die einen Durchmesser von 40 mm und eine Länge von 0 2000 mm hatte. Sie enthielt Gewebepackungen aus Edelstahl mit einer Oberfläche von 1000 m²/m³ . Die Füllung der Kolonne entsprach 40 theoretischen Böden. Am Kolonnenkopf befand sich ein Hauptkondensator, der mit Wasser einer Temperatur von 40°C betrieben wurde. Der Nachkondensator am Kolonnenkopf wurde mit Sole gekühlt, die eine Temperatur von 0°C hatte. Man dosierte im unteren Drittel der Kolonne 350 g/h rohes N-Vinylformamid, das auf eine Temperatur von 75°C vorgewärmt worden war und 15 Gew.-% Formylalaninnitril enthielt. Zum Verdampfer am Kolonnensumpf wurden 100 g/h flüssiges Formamid gepumpt. Die Verdampferleistung wurde so geregelt, daß die Temperatur im unteren Drittel der Kolonne möglichst konstant blieb. Der Druck betrug am Kopf der Kolonne 2 mbar. Am Hauptkondensator fielen 500 g/h Kondensat an, das vollständig als Rücklauf zur Kolonne geleitet wurde.

Am oberen Viertel der Kolonne erhielt man als flüssigen Seitenabzug 290 g/h N-Vinylformamid in einer Reinheit von mehr als 99 Gew.-%. Der Anteil an Formamid betrug in diesem Seitenabzug 0,08 Gew.-%. Diejenigen Stoffe, die im Hauptkondensator nicht kondensiert werden konnten, wurden im Nachkondensator niedergeschlagen und ausgetragen. Im stationären Betrieb fielen im Sumpf bei einem Druck von 10 mbar (gemessen im Sumpf) und einer 5 Temperatur von 95°C ca. 160 g/h Produkt an. Das Sumpfprodukt enthielt weniger als 0,1 Gew.-% N-Vinylformamid.

### Beispiel 3

0 Für die kontinuierliche Destillation von N-Vinyl-2-pyrrolidon verwendete man eine Kolonne von 40 mm und einer Länge von 2000 mm. Sie enthielt Gewebepackungen aus Edelstahl mit einer Oberfläche von 1000 m²/m³. Die Füllung der Kolonne entsprach etwa 40 theoretischen Böden. Am Kolonnenkopf befand sich ein Hauptkondensator, der mit Wasser einer Temperatur von 50°C betrieben wurde. Der Nachkondensator, der oberhalb des Hauptkondensators angeordnet war, wurde mit Sole gekühlt, die eine Temperatur von 0°C hatte. In das untere Drittel der Kolonne pumpte man kontinuierlich 300 g/h N-Vinylpyrrolidon, das auf eine Temperatur von 75°C vorgewärmt worden war. Zum Verdampfer am Kolonnenkopf wurden 100 g/h flüssiges Formamid kontinuierlich dosiert. Die Verdampferleistung wurde so geregelt, daß die Temperatur im untern Drittel der Kolonne möglichst konstant blieb. Der Druck betrug am Kopf der Kolonne 2 mbar. Am Hauptkondensator fielen 600 g/h Kondensat an, das vollständig als Rücklauf zur Kolonne geleitet wurde. Am oberen Viertel der Kolonne erhielt man als flüssigen Seitenabzug 250 g/h N-Vinylpyrrolidon mit einer Reinheit von mehr als 99 Gew.-% und einem Gehalt an Formamid von 0,06 Gew.-%. Diejenigen Stoffe, die im Hauptkondensator nicht kondensiert werden konnten, wurden im Nachkondensator niedergeschlagen und separat ausgetragen. Im stationären Betrieb fielen im Sumpf bei einem Druck von 10 mbar (gemessen im Sumpf der Kolonne) und einer Temperatur von 95°C ca. 185 g/h Produkt an. Das Sumpfprodukt enthielt weniger als 0,1 Gew.-% N-Vinylpyrrolidon.

## Patentansprüche

1. Verfahren zur kontinuierlichen Destillation von N-Vinylcarbonsäureamiden in Gegenwart von Formamid in einer Kolonne unter einem Druck, der am Kopf der Kolonne 0,1 bis 30 mbar beträgt, dadurch gekennzeichnet, daß man
- die N-Vinylcarbonsäureamiden getrennt von Formamid kontinuierlich in flüssiger Form in den unteren Teil der Kolonne bis zur Mitte der Kolonne zuführt,
- Formamid mit Hilfe eines Verdampfers am Kolonnensumpf verdampft und zusammen mit Bestandteilen, die höher als die N-Vinylcarbonsäureamiden sieden, aus dem Kolonnensumpf austrägt und durch Zugabe von frischem Formamid ersetzt,
- die N-Vinylcarbonsäureamiden in einem Seitenabzug im oberen Drittel der Kolonne abnimmt, wobei man die Destillation so steuert, daß die Monomeren weniger als 5 Gew.-% Formamid enthalten, und
- am Kopf der Kolonne einen Produktstrom abnimmt, der Bestandteile enthält, die einen niedrigeren Siedepunkt als die N-Vinylcarbonsäureamiden haben.

2. Verfahren nach Anspruch 1, dadurch gekennzeichnet, daß im Seitenabzug der Kolonne N-Vinylcarbonsäureamide mit einem Gehalt von weniger als 0,1 Gew.-% Formamid abgenommen werden.

3. Verfahren nach Anspruch 1 oder 2, dadurch gekennzeichnet, daß im Seitenabzug der Kolonne N-Vinylcarbonsäureamide abgenommen werden, die frei von Formamid sind.

4. Verfahren nach einem der Ansprüche 1 bis 3, dadurch gekennzeichnet, daß man als N-Vinylcarbonsäureamid N-Vinylformamid einsetzt.

5. Verfahren nach einem der Ansprüche 1 bis 3, dadurch gekennzeichnet, daß man als N-Vinylcarbonsäureamid N-Vinylpyrrolidon einsetzt.

## Claims

1. A process for the continuous distillation of N-vinylcarboxamides in the presence of formamide in a column under a pressure of 0.1 to 30 mbar at the top, wherein
- the N-vinylcarboxamides are continuously fed in liquid form, separately from formamide, into the lower part of the column up to the middle of the column,
- Formamide is vaporized by means of a vaporizer at the bottom of the column and, together with constituents having higher boiling points than the N-vinylcarboxamides is discharged from the bottom of the column and replaced by addition of fresh formamide,
- the N-vinylcarboxamides are taken off at a side offtake in the upper third of the column, with the distillation being controlled such that the monomers contain less than 5 % by weight of formamide, and
- a product stream containing constituents which have a lower boiling point than the N-vinylcarboxamides is taken off at the top of the column.

2. A process as claimed in claim 1, wherein N-vinylcarboxamides having a formamide content of less than 0.1 % by weight are D taken off at the side offtake of the column.

3. A process as claimed in claim 1 or 2, wherein N-vinylcarboxamides which are free of formamide are taken off at the side offtake of the column.

4. A process as claimed in any of claims 1 to 3, wherein the N-vinylcarboxamide used is N-vinylformamide.

5. A process as claimed in any of claims 1 to 3, wherein the N-vinylcarboxamide used is N-vinylpyrrolidone.

## Revendications

1. Procédé de distillation continue d'amides d'acide N-vinylcarboxylique en présence de formamide dans une colonne, sous une pression qui est à la tête de la colonne de 0,1 à 30 mbars, caractérisé en ce que
- on amène les amides d'acide N-vinylcarboxylique en continu, séparément du formamide, sous forme liquide dans la partie inférieure de la colonne jusqu'au milieu de la colonne,
- on évapore du formamide à l'aide d'un évaporateur en bas de colonne et on l'évacue par le bas de colonne conjointement avec des éléments qui ont un point d'ébullition supérieur aux amides d'acide N-vinylcarboxylique et on le remplace par addition de formamide frais,
- on prélève les amides d'acide N-vinylcarboxylique dans une sortie latérale dans le tiers supérieur de la colonne, en dirigeant la distillation de façon que les monomères contiennent moins de 5% en poids de formamide, et
- à la tête de la colonne on prélève un courant de produit qui contient des composants qui ont un point d'ébullition inférieur aux amides d'acide N-vinylcarboxylique.

2. Procédé suivant la revendication 1, caractérisé en ce que, dans la sortie latérale de la colonne, on prélève des amides d'acide N-vinylcarboxylique ayant une teneur en formamide inférieure à 0,1% en poids.

3. Procédé suivant l'une des revendications 1 et 2, caractérisé en ce que, dans la sortie latérale de la colonne, on prélève des amides d'acide N-vinylcarboxylique qui sont exempts de formamide.

4. Procédé suivant l'une des revendications 1 à 3, caractérisé en ce qu'on met en oeuvre, comme amide d'acide N-vinylcarboxylique, du N-vinylformamide.

5. Procédé suivant l'une des revendications 1 à 3, caractérisé en ce que, comme amide d'acide N-vinylcarboxylique, on met en oeuvre de la N-vinylpyrrolidone.
